# EUROPEAN PATENT SPECIFICATION

(11) **EP 4 103 002 B1**
(45) Date of publication and mention of the grant of the patent: **27.11.2024**
(21) Application number: 21703744.9
(22) Date of filing: 12.02.2021
(51) Int. Cl.: A24F 40/40, A24D 1/20, A24F 40/20, A24F 40/51

(54) **SMOKING SUBSTITUTE SYSTEM**
RAUCHERSATZSYSTEM
SYSTÈME DE SUBSTITUTION DU TABAC

(30) Priority: 14.02.2020 EP 20157501; 14.02.2020 EP 20157500
(43) Date of publication of application: 21.12.2022
(60) Divisional application: 24205673.7
(73) Proprietor: Imperial Tobacco Limited, Bristol, BS3 2LL (GB)
(72) Inventor: FERRIE, Kate, Liverpool Merseyside L24 9HP (GB); SHENTON, Ross, Liverpool m L24 9HP (GB); LOMAS, Pete, Liverpool Merseyside L24 9HP (GB)
(74) Representative: Mewburn Ellis LLP
(86) International application number: PCT/EP2021/053455
(87) International publication number: WO 2021/160803

(56) References cited:
- EP-A1- 3 192 382
- WO-A1-2014/195687
- WO-A1-2019/170893
- WO-A1-2019/185745

## Description

### TECHNICAL FIELD

The present invention relates to a smoking substitute device having a closure to close a cavity configured for receipt of at least a portion of a consumable. The invention also relates to a smoking substitute system comprising a device and an aerosol-forming article.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumable") that are designed to resemble a traditional cigarette and are cylindrical in form with a mouthpiece at one end.

The popularity and use of smoking substitute systems has grown rapidly in the past few years. These systems assist habitual smokers wishing to quit tobacco smoking.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

WO2019170893A1, WO2019185745A1, and EP3192382A1 disclose heating devices comprising different designs of closures to prevent contamination. WO2014195687A1 discloses a closure to cover a container for a smoking substitute system.

There may be a need for improved design of smoking substitute systems, in particular HT smoking substitute systems, to enhance the user experience and improve the function of the HT smoking substitute system.

The present disclosure has been devised in the light of the above considerations.

### SUMMARY OF THE INVENTION

At their most general, aspects of the present disclosure relate to a heat-not-burn device having a closure for covering an opening of a cavity configured for receipt of at least a portion of a consumable. Other aspects relate to the detection of the presence of an aerosol-forming article in a cavity of a smoking substitute device.

According to a first aspect of the present invention, there is provided a smoking substitute device as defined by claim 1.

In other words, the system of the first aspect provides for covering or hiding from external influence the consumable opening or cavity of a smoking substitute device like a heated tobacco device. In particular, the present invention is designed to provide a means of covering the opening when no consumable is inserted into the device. The closure comprises a channel and is free to rotate about an axis and may be rotated to align with a cavity, which may be channel-shaped as well, allowing the device to be used by allowing a consumable to be inserted. The opening in the closure, an upper channel, is a complete thru hole, while the lower channel may contain the heating element, e.g. being a cavity in the body of the smoking substitute device.

Thereby, the present invention may provide a simple way of covering the consumable receiving opening/cavity, to reduce the chance that dirt or debris enters, ingress or egress from the device, in particular the cavity. The closure may be intuitive in use and easy for the user of a smoking substitute device to perform and may also be user to switch on and/or off the smoking substitute device.

The closure may be provided with a handle external to the body of the device for allowing a user to move the closure between the first position and the second position. In some embodiments, the handle forms part of the closure or is connected to the closure. In some embodiments, the handle comprises a rotatable handle. In some embodiments, the handle comprises a movable part outside the body of the device and a connector passing through an outer wall of the device and attached to the closure within the body of the device. Movement of the movable part by the user allows the user to control the position of the closure. In some embodiments the connector is housed within a slot in the outer wall of the device and slidable along the slot by movement of the movable part.

Providing a heat-not-burn device comprising a closure which covers the opening of the cavity helps prevent the entry of dust/dirt particles into the cavity. Further, the presence of the closure may prevent particles of aerosol-forming-substrate (e.g. tobacco) which have accumulated within the device from falling out of the cavity of the device. Additionally, a closure which is substantially concealed when in the second position provides a more ergonomic design to improve the user experience and prevent the closure interfering with the use of the device.

In the second position of the closure, an aperture is provided at the opening of sufficient size to permit a consumable to be inserted into the device. In other words, in the second position the closure does not cover the opening, or only covers the opening only to the extent that a consumable may still be inserted into the opening and into the cavity of the device.

In some embodiments, the first position is a terminal position along the path of travel of the closure, and the second position is a terminal position along the path of travel of the closure. Thus, the closure may move along a path of travel which terminates at each end in the first and second positions respectively.

According to a preferred embodiment of the present invention the device may comprise means to hold the closure in one or more of the first position and the second position.

According to a further preferred embodiment of the present invention, the means to hold the closure may be at least one means out of the group consisting of a detent comprising a raised feature on a surface of the device body, a magnet or a spring.

In some embodiments, the means to hold the closure comprises an interaction between the closure and a part of the body of the device which occurs at or close to the first and/or second position. In some embodiments, the means to hold the closure comprises a detent comprising a raised feature on a surface of the device body and/or the closure. In some embodiments, the means to hold the closure comprises an interference fit provided between the closure and the body of the device when in the first and/or second positions, wherein the interference fit is removed as the closure moves away from the first and/or second position to facilitate movement between the positions.

According to a further preferred embodiment of the present invention, the closure may be provided with the handle external to the body of the device for allowing a user to move the closure between the first position and the second position.

According to a further preferred embodiment of the present invention, the closure may be a made of flexible material.

In this way, the closure is able to bend and flex when moved, to facilitate its accommodation within the body of the device.

According to a further preferred embodiment of the present invention, the smoking substitute device may further comprise a sensor for detecting a position of the closure, a heater for heating the consumable when received in the cavity and a controller configured to receive a signal from the sensor, indicative of a position of the closure, and to control the heater in response to the received signal.

According to a further preferred embodiment of the present invention, the sensor may be configured to generate a signal upon detecting that the closure is in the first position, and wherein the controller may deactivate the heater based on the received signal.

In some embodiments, the controller prevents activation of the heater when the closure is in the first position. In some embodiments, the controller permits activation of the heater when the closure is in the second position. In some embodiments, the controller activates the heater when the closure is moved into the second position, e.g. by a user operating the handle. In this way, the heater cannot be activated when the closure is "closed" and/or can be activated/is automatically activated when the closure is "open". This provides a safer and more efficient device since accidental activation of the heater e.g. in a pocket or bag is prevented, which saves battery life and is safer. When the user opens the closure, the controller then permits the activation of the heater (e.g. by an appropriate input on a user interface) or automatically actives the heater.

Thus, the sensor may be configured to generate a signal upon detecting that the closure is in the first position. In some embodiments, the controller then deactivates the heater, based on the received signal. In this way, unnecessary power supply to the heater is avoided when the device is not in use.

According to a further preferred embodiment of the present invention claims, the closure may comprise biasing means which urge the closure into one or both of the first position and the second position.

According to a further preferred embodiment of the present invention, the biasing means may comprise a magnet or spring.

In this way, the risk of accidental movement of the closure away from the first position or away from the second position is reduced. In some embodiments, the biasing means comprises a magnet or spring. For example, the closure and the body of the device may each comprise a magnet, between which a force of attraction exists to hold the closure in position until the user overcomes the force. Alternatively, the closure may be spring-loaded, wherein one or more springs bias the closure into one or more of the first and second positions. In some embodiments, the biasing means urge the closure into the first position when the closure is positioned at a position intermediate the first and second positions.

According to a further preferred embodiment of the present invention, the closure may be interposed between an opening to the cavity and a rod heater, wherein the rod heater may be disposed within the cavity along a longitudinal axis of the body.

Conveniently, the rod heater is disposed along a longitudinal axis of the body. In some embodiments, the closure when in the first position is set back from the opening in the body of the device, such that a recess is provided at the opening of the cavity when the closure is in the first position (closed).

According to further arrangements, which may fall outside the scope of the invention, the closure may be at least one of a swivelling closure, a swinging closure, a sliding closure..

In some embodiments, the closure comprises a swivelling or rotating closure, such as a ball valve. Such a ball valve may comprise a body of circular or substantially circular cross section which is rotatable, defining a bore passing through the body, such that rotation of the body brings the bore of the body and the cavity of the device into alignment (second position) for insertion of a consumable. When the body is rotated away from this alignment (into a first position) the opening of the cavity in the device is effectively covered. The ball valve may comprise a cylindrical body defining a bore passing through the body in a direction perpendicular to the primary rotational axis of the cylinder. In this way, the user rotates the cylinder until the bore and cavity are in alignment (in a second position) to facilitate insertion of a consumable into the device, and rotates the cylinder so that the bore and cavity are out of alignment (in a first position) to effectively cover or restrict entry to the opening to the cavity. In some embodiments the ball valve structure is contained within the body of the device such that it is substantially concealed within the body.

In some arrangements which fall outside the scope of the invention, the closure comprises a swinging closure, such as a concealed trap door within the device body. For example, in some arrangements that fall outside of the scope of claim 1, the closure may comprise a hinged sheet of material which is biased into the first position (closed) in which the sheet covers the opening, wherein when force is applied to the sheet in a direction into the device to overcome the bias, the sheet swings via the hinge into the second position (open), allowing insertion of a consumable into the cavity. In this way, the user is able to open the closure simply by pressing the end of a consumable against the sheet, into the device, which pushes the closure away from the opening to allow the consumable to pass into the device. In some embodiments, the trap door is biased into the first position (closed). For example, the trap door may be spring-loaded.

In further arrangements that fall outside the scope of the invention, the closure comprises a sliding closure, such as a planar sheet of material which in the first position extends across the opening and in the second position in retracted within the body of the device. In some embodiments, the sheet resides within a slot adjacent to the cavity when in the second position, and slides out of the slot and across the opening when moved into the first position. In some of these arrangements the planar sheet of material is flexible. In this way, when the sheet is retracted into the second position it may bend or flex to conform to the internal structure of the body of the device to be more easily accommodated.

According to a second aspect of the present disclosure there is provided a smoking substitute device comprising a cavity for receiving a consumable, and a detection element for detecting whether a consumable is arranged in the cavity, wherein the cavity comprises at least one deformable wall and wherein the at least one deformable wall is deformable by the insertion of a consumable such that the at least one deformable wall assumes a different position within the smoking substitute device with a consumable inserted in the cavity than with no consumable inserted in the cavity.

By providing a smoking substitute device comprising a deformable wall, the device may detect the insertion of a consumable in the cavity of the smoking substitute device and/or detect the presence of a consumable in the cavity of the smoking substitute device to prepare the use substantially without any user input, and may subsequently commence operation substantially without any user input.

In other words, the second aspect of the present disclosure provides for registering of a consumable insertion into the smoking substitute device by a user. Thus, the heated tobacco device is adapted to register the fact that a consumable has been inserted, either directly or by a controller. Further, this functionality may operate as a safety switch, possibly including a triggering of heating when insertion of a consumable is detected to the device so to render it unnecessary for a user to press a button to initiate heating. A detection element, like a trigger or an electrical contact, e.g. a combination of metal contacts establishing a conductive connection, may allow the smoking substitute device to register that a consumable is inside. This also allows user feedback on insertion of the device, meaning providing an information to a user that a consumable is (properly) inserted. This may also avoid that the user tries to insert a consumable with excess force due to lack of feedback, thereby possibly damaging the smoking substitute device or parts thereof.

The term "deformable" is intended to refer to an element that changes its shape, form and/or position relative to other elements. E.g. in case of a deformable wall of the cavity, a deformable wall may refer to a wall of the cavity changing its shape or form, e.g. being bent or twisted, in particular by the insertion and/or removal of a consumable. On the same token, deformable may merely mean that the position of the deformable wall in the interior of the smoking substitute device relative to other elements may change, without actually changing shape or form of the deformable wall. In other words, the deformable wall is shifted in its position relative to other elements, with a consumable inserted in the cavity and with no consumable inserted in the cavity.

Optional features will now be set out. These are applicable singly or in any combination with any aspect.

According to a preferred embodiment of the present disclosure the detection of a consumable present in the cavity may be triggered by the deformation of the at least one deformable wall.

According to a further preferred embodiment of the present disclosure the detection element may be arranged adjacent to the at least one deformable wall of the cavity, in particular to detect the deformation of the at least one deformable wall with a consumable present in the cavity.

In other words, the deformation of the wall may be such that the deformation, which may coincide with a shift in position or a relocation of the deformable wall within the interior of the smoking substitute device, triggers the detection by said movement. The deformable wall may thus shift its position so to press against the detection element, thereby triggering the detections element. The detection element may detect this shift, e.g. by a change in a force or pressure acting on the detection element. Likewise, the detection element may be an optical detection element, detecting a change in illumination resulting from the shift in position of the deformable wall. Beneficial for the proper detection is arranging the detection close to or adjacent to the deformable wall, so to be able to detect even a comparably small deformation or relocation/shift in position.

According to a further preferred embodiment of the present disclosure, the at least one deformable wall, when a consumable is inserted in the cavity, may trigger the detection element by closing an electric circuit.

According to a further preferred embodiment of the present disclosure, the detection element may be a switch and the deformation of the wall may trigger the switch and/or wherein the detection element may be an electrical connection element and the deformation of the wall may close or open an electrical circuit.

In other words, the deformation of the deformable wall occurring when a consumable is inserted into the cavity of the smoking substitute device may press against the detection element thereby closing an electric circuit. Alternatively, the detection element may be embodied as an element comprising a plurality of electrical contacts, with the deformable wall comprising itself a conductive element arranged adjacent to the detection element, so that the deformation, e.g. the shift in position of the deformable wall, brings the conductive element of the deformable wall in contact with the detection element, in particular the electrical contacts thereof, thereby closing the circuit.

According to a further preferred embodiment of the present disclosure, the detection element may be adapted for activation and/or deactivation of an operation of the smoking substitute device, in particular may be adapted for activation and/or deactivation of a heating element of the smoking substitute device.

According to a further preferred embodiment of the present disclosure, the smoking substitute device may be commencing operation upon insertion of a consumable substantially without further user interaction with the smoking substitute device, and/or wherein the smoking substitute device may be halting operation upon removal of a consumable substantially without further user interaction with the smoking substitute device.

In other words, upon detection of the insertion of a consumable in the cavity of the smoking substitute device, the operation, e.g. the heating of the consumable, may commence, substantially without further user interaction. This may be realized by the closing of an electric circuit by the detection element, thereby providing energy to the heating element, a controller of the smoking substitute device or the like. In particular, a further press of a button to switch on the smoking substitute device or to activate heating may not be required. Likewise, the detection of the removal of the consumable may halt operation, e.g. disconnect the power from the heating element, the controller or the like. The detection of presence or absence of a consumable may equally be done by the controller, obtaining a signal from the detection element, signalling the presence or absence of ta consumable in the cavity.

According to a further preferred embodiment of the present disclosure the removal of a consumable may provide an emergency shutdown functionality for the smoking substitute device.

E.g. a removal of the consumable from the cavity may be detected, and a controller may substantially immediately halt the operation of the smoking substitute device, e.g. providing energy to the heating element. Likewise, the removal itself may open an electric circuit for providing energy to the heating element. Thus, there is provided an easy and intuitive way of stopping the operation of the smoking substitute device, e.g. in case of malfunction.

According to a further preferred embodiment of the present disclosure, the smoking substitute device may comprise at least one further detection element for detecting whether a consumable is arranged in the cavity, wherein the cavity may comprise at least one further deformable wall, wherein the at least one further deformable wall may be deformable by the insertion of a consumable such that the at least one deformable wall assumes a different position within the smoking substitute device with a consumable inserted in the cavity than with no consumable inserted in the cavity.

According to a further preferred embodiment of the present disclosure, the presence of the consumable in the cavity may be detected by the detection element and the at least one further detection element collectively.

According to a further preferred embodiment of the present disclosure, the at least one further detection element may be a further switch and the deformation of the at least one further deformable wall triggers the further switch; and/or wherein the at least one further detection element may be a further electrical connection element and the deformation of the wall closes or opens an electrical circuit, in particular wherein the collective operation of the detection element and the at least one further detection element may provide the detection of the insertion of the consumable, further in particular by closing or opening an electrical circuit comprising the detection element and the at one further detection element.

Providing at least two detections elements may allow a redundant detection, e.g. having more than one detection element and thus detection result, in case one detection element malfunctions. Further, providing more than one detection element may allow to detect the proper insertion in more than one degree of freedom. E.g. one detection element may determine an insertion with regard to the width of the cavity or consumable, while the other may detect the depth of the insertion, e.g. how deep a consumable is inserted into the cavity. Only when the detections elements determine that a consumable is inserted correctly regarding all relevant degrees of freedom, an overall correct insertion is determined, resulting in an according reaction, like activation of the heating element. Also, the plurality of detection element may operate collectively. This means that only a single determination is effected, when a consumable is inserted correctly in relation to all detection elements.

E.g. in case of two detections elements, both may be electrical connection elements. The deformable walls may contact the detection elements and closes an electrical connection between the detection elements. The closing of the electrical connection may be provided by a conductive path arranged e.g. on the deformable walls, that closes the electrical circuit by connecting to the two electrical connection elements.

According to a further preferred embodiment of the present disclosure, the cavity and/or at least one of the deformable walls may be suspended in the smoking substitute device by at least one spring element, and wherein the at least one spring element may provide at least partially a relocation of the at least one of the deformable walls for assuming a different position within the smoking substitute device with a consumable inserted in the cavity than with no consumable inserted in the cavity.

According to a further preferred embodiment of the present disclosure, the at least one spring element may allow a relocation of the cavity within the smoking substitute device with the insertion of a consumable into the cavity, so that an electronic circuit is closed when the consumable is inserted into the cavity.

The cavity, e.g. a heating chamber of a smoking substitute device may be mounted on spring elements that, as the user inserts the consumable, allow a stretching, or generally a relocation of the deformable walls, allowing the electrical connection elements to close an electric circuit.

According to a further preferred embodiment of the present disclosure, the detection of a consumable present in the cavity may be triggered by the deformation of the at least one deformable wall, in particular by the at least one deformable wall closing an electric circuit.

The device may comprise an elongate body. An end of the elongate body may be configured for engagement with an aerosol-forming article. For example, the body may be configured for engagement with a heated tobacco (HT) consumable (or heat-not-burn (HNB) consumable). The terms "heated tobacco" and "heat-not-burn" are used interchangeably herein to describe a consumable that is of the type that is heated rather than combusted (or are used interchangeably to describe a device for use with such a consumable). The device may comprise a cavity that is configured for receipt of at least a portion of the consumable (i.e. for engagement with the consumable). The aerosol-forming article may be of the type that comprises an aerosol former (e.g. carried by an aerosol-forming substrate).

The device may comprise a heater for heating the aerosol-forming article. The heater may comprise a heating element, which may be in the form of a rod that extends from the body of the device. The heating element may extend from the end of the body that is configured for engagement with the aerosol-forming article.

The heater (and thus the heating element) may be rigidly mounted to the body. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al₂O₃. The core of the heating element may have a diameter of 1.8 mm to 2.1 mm, e.g. between 1.9 mm and 2 mm. The heating element may comprise an outer layer (e.g. an outer ceramic layer) comprising Al₂O₃. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into an aerosol-forming article (e.g. a HT consumable) when an aerosol-forming article is received in the cavity. In that respect, a distal end (i.e. distal from a base of the heating element where it is mounted to the device) of the heating element may comprise a tapered portion, which may facilitate insertion of the heating element into the aerosol-forming article. The heating element may fully penetrate an aerosol-forming article when the aerosol-forming article is received in the cavity. That is, the entire length, or substantially the entire length, of the heating element may be received in the aerosol-forming article.

The heating element may have a length that is less than, or substantially the same as, an axial length of an aerosol-forming substrate forming part of an aerosol-forming article (e.g. a HT consumable). Thus, when such an aerosol-forming article is engaged with the device, the heating element may only penetrate the aerosol-forming substrate, rather than other components of the aerosol-forming article. The heating element may penetrate the aerosol-forming substrate for substantially the entire axial length of the aerosol forming-substrate of the aerosol-forming article. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the aerosol-forming article is received in the cavity, the heating element may surround a portion of the aerosol-forming article (i.e. so as to heat that portion of the aerosol-forming article). In particular, the heating element may surround an aerosol forming substrate of the aerosol-forming article. That is, when an aerosol-forming article is engaged with the device, the aerosol forming substrate of the aerosol-forming article may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the aerosol-forming article. The wall (or walls) of the cavity may be formed from a thermally conductive material (e.g. a metal) to allow heat conduction from the heating element to the aerosol-forming article. Thus, heat may be conducted from the heating element, through the cavity wall (or walls), to the aerosol-forming substrate of an aerosol-forming article received in the cavity.

In some embodiments the device may comprise a cap disposed at the end of the body that is configured for engagement with an aerosol-forming article. Where the device comprises a heater having a heating element, the cap may at least partially enclose the heating element. The cap may be moveable between an open position in which access is provided to the heating element, and a closed position in which the cap at least partially encloses the heating element. The cap may be slideably engaged with the body of the device, and may be slideable between the open and closed positions.

The cap may define at least a portion of the cavity of the device. That is, the cavity may be fully defined by the cap, or each of the cap and body may define a portion of the cavity. Where the cap fully defines the cavity, the cap may comprise an aperture for receipt of the heating element into the cavity (when the cap is in the closed position). The cap may comprise an opening to the cavity. The opening may be configured for receipt of at least a portion of an aerosol-forming article. That is, an aerosol-forming article may be inserted through the opening and into the cavity (so as to be engaged with the device).

The cap may be configured such that when an aerosol-forming article is engaged with the device (e.g. received in the cavity), only a portion of the aerosol-forming article is received in the cavity. That is, a portion of the aerosol-forming article (not received in the cavity) may protrude from (i.e. extend beyond) the opening. This (protruding) portion of the aerosol-forming article may be a terminal (e.g. mouth) end of the aerosol-forming article, which may be received in a user's mouth for the purpose of inhaling aerosol formed by the device.

The device may comprise a power source or may be connectable to a power source (e.g. a power source separate to the device). The power source may be electrically connectable to the heater. In that respect, altering (e.g. toggling) the electrical connection of the power source to the heater may affect a state of the heater. For example, toggling the electrical connection of the power source to the heater may toggle the heater between an on state and an off state. The power source may be a power store. For example, the power source may be a battery or rechargeable battery (e.g. a lithium ion battery).

The device may comprise an input connection (e.g. a USB port, Micro USB port, USB-C port, etc.). The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to the heater (for providing power to the heater). Hence, in some forms, the input connection may form at least part of the power source of the device.

Where the power source comprises a rechargeable power source (such as a rechargeable battery), the input connection may be used to charge and recharge the power source.

The device may comprise a user interface (UI). In some embodiments the UI may include input means to receive operative commands from the user. The input means of the UI may allow the user to control at least one aspect of the operation of the device. In some embodiments the input means may comprise a power button to switch the device between an on state and an off state.

In some embodiments the UI may additionally or alternatively comprise output means to convey information to the user. In some embodiments the output means may comprise a light to indicate a condition of the device (and/or the aerosol-forming article) to the user. The condition of the device (and/or aerosol-forming article) indicated to the user may comprise a condition indicative of the operation of the heater. For example, the condition may comprise whether the heater is in an off state or an on state. In some embodiments, the UI unit may comprise at least one of a button, a display, a touchscreen, a switch, a light, and the like. For example, the output means may comprise one or more (e.g. two, three, four, etc.) light-emitting diodes ("LEDs") that may be located on the body of the device.

The device may further comprise a puff sensor (e.g. airflow sensor), which form part of the input means of the Ul. The puff sensor may be configured to detect a user drawing on an end (i.e. a terminal (mouth) end) of the aerosol-forming article. The puff sensor may, for example, be a pressure sensor or a microphone. The puff sensor may be configured to produce a signal indicative of a puff state. The signal may be indicative of the user drawing (an aerosol from the aerosol-forming article) such that it is e.g. in the form of a binary signal. Alternatively or additionally, the signal may be indicative of a characteristic of the draw (e.g. a flow rate of the draw, length of time of the draw, etc).

The device may comprise a controller, or may be connectable to a controller that may be configured to control at least one function of the device. The controller may comprise a microcontroller that may e.g. be mounted on a printed circuit board (PCB). The controller may also comprise a memory, e.g. non-volatile memory. The memory may include instructions, which, when implemented, may cause the controller to perform certain tasks or steps of a method. Where the device comprises an input connection, the controller may be connected to the input connection.

The controller may be configured to control the operation of the heater (and e.g. the heating element). Thus, the controller may be configured to control vaporisation of an aerosol forming part of an aerosol-forming article engaged with the device. The controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater.

In some embodiments, where the device comprises a Ul, the controller may be operatively connected to one or more components of the UI. The controller may be configured to receive command signals from an input means of the UI. The controller may be configured to control the heater in response to the command signals. For example, the controller may be configured to receive "on" and "off" command signals from the UI and, in response, may control the heater so as to be in a corresponding on or off state.

The controller may be configured to send output signals to a component of the Ul. The UI may be configured to convey information to a user, via an output means, in response to such output signals (received from the controller). For example, where the device comprises one or more LEDs, the LEDs may be operatively connected to the controller. Hence, the controller may configured to control the illumination of the LEDs (e.g. in response to an output signal). For example, the controller may be configured to control the illumination of the LEDs according to (e.g. an on or off) state of the heater.

Where the device comprises a sensor (e.g. a puff/airflow sensor), the controller may be operatively connected to the sensor. The controller may be configured to receive a signal from the sensor (e.g. indicative of a condition of the device and/or engaged aerosol-forming article). The controller may be configured to control the heater, or an aspect of the output means, based on the signal from the sensor.

The device may comprise a wireless interface configured to communicate wirelessly (e.g. via Bluetooth (e.g. a Bluetooth low-energy connection) or WiFi) with an external device. Similarly, the input connection may be configured for wired connection to an external device so as to provide communication between the device and the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device. Alternatively or additionally, an external device may respond to a signal received from the device (e.g. from the controller of the device).

According to the invention, as defined in claim 11, there is provided a smoking substitute system comprising a smoking substitute device according to claim 1, and an aerosol-forming article. The aerosol-forming article may comprise an aerosol-forming substrate at an upstream end of the aerosol-forming article. The article may be in the form of a smoking substitute article, e.g. heated tobacco (HT) consumable (also known as a heat-not-burn (HNB) consumable).

All the optional features of the closure described above in the context of the first aspect apply equally to the third aspect. All the optional features of the detection element and/or the at least one deformable wall described above in the context of the second aspect also apply equally to the third aspect.

As used herein, the terms "upstream" and "downstream" are intended to refer to the flow direction of the vapour/aerosol i.e. with the downstream end of the article/consumable being the mouth end or outlet where the aerosol exits the consumable for inhalation by the user. The upstream end of the article/consumable is the opposing end to the downstream end.

The aerosol-forming substrate is capable of being heated to release at least one volatile compound that can form an aerosol. The aerosol-forming substrate may be located at the upstream end of the article/consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10 mm e.g. between 6 and 9 mm or 6 and 8 mm e.g. around 7 mm. It may have an axial length of between 10 and 15 mm e.g. between 11 and 14 mm such as around 12 or 13 mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the article/consumable may comprise an aerosol-cooling element which is adapted to cool the aerosol generated from the aerosol-forming substrate (by heat exchange) before being inhaled by the user.

The article/consumable may comprise a spacer element that defines a space or cavity between the aerosol-forming substrate and the downstream end of the consumable. The spacer element may comprise a cardboard tube. The spacer element may be circumscribed by the (paper) wrapping layer.

According to the invention as defined in claim 12, there is provided a method of operating a smoking substitute device according to claim 1, the method comprising the steps of determining a position of a closure for covering a cavity of the device into which an aerosol generating consumable (202) is received in use and controlling a heater of the device based on the determined position of the closure.

According to a preferred embodiment of the present invention, the method may comprise determining, by a sensor associated with the controller, the position of the closure, wherein the controller deactivates the heater based on a signal received from the sensor when the closure is in a first position where the closure covers a cavity in the device.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute system with the consumable disengaged from the device;
Figure 2C is a section view of the consumable of the first embodiment of the smoking substitute system;
Figure 2D is a detailed view of an end of the device of the first embodiment of the smoking substitute system;
Figure 2E is a section view of the first embodiment of the substitute smoking system;
Figure 3A to 3E illustrate schematic perspective views of an embodiment of the substitute smoking system according to the first aspect of the invention comprising a closure;
Figure 4A is a detailed view of an embodiment of the smoking substitute device according to the second aspect of the invention with no consumable inserted in the device; and
Figure 4B is a detailed view of the smoking substitute device of Figure 4A, showing a consumable inserted in the device

### DETAILED DESCRIPTION OF THE INVENTION

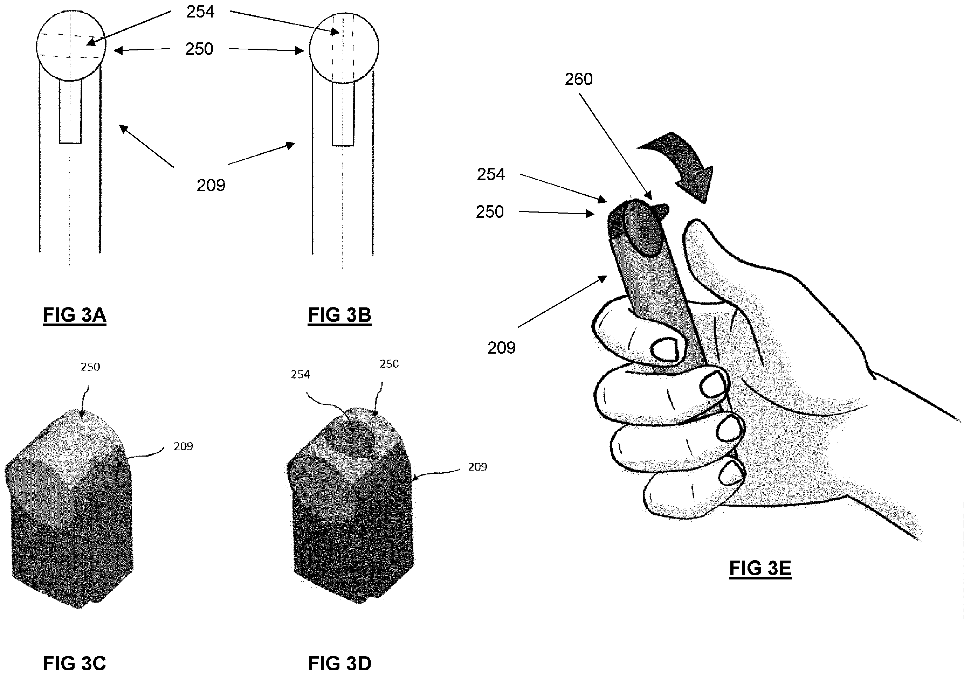

Aspects and embodiments of the present invention will now be discussed with reference to the accompanying figures, wherein figures 3A-3E show a device which is fully in accordance with the present invention as defined in claims 1 and 12. Further aspects and embodiments will be apparent to those skilled in the art.

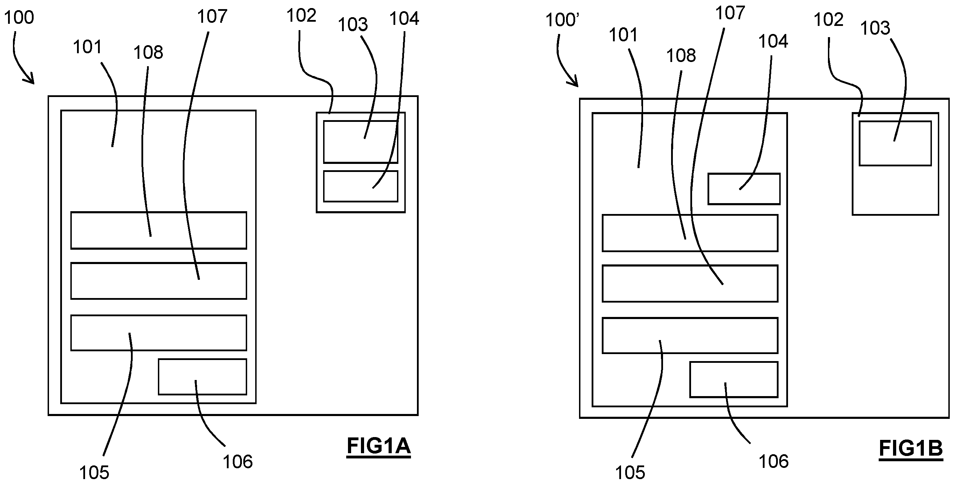

Figure 1A is a schematic providing a general overview of a smoking substitute system 100. The system 100 includes a substitute smoking device 101 and an aerosol-forming article in the form of a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the consumable 102 and is configured to heat the aerosol former 103. In this variation, the heater 104 is electrically connectable to the power source 105, for example, when the consumable 102 is engaged with the device 101. Heat from the heater 104 vaporises the aerosol former 103 to produce a vapour. The vapour subsequently condenses to form an aerosol, which is ultimately inhaled by the user.

The system 100 further comprises a power source 105 that forms part of the device 101. In other embodiments the power source 105 may be external to (but connectable to) the device 101. The power source 105 is electrically connectable to the heater 104 such that it is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising a connector 106 (e.g. in the form of a USB port, Micro USB port, USB-C port, etc.). The connector 106 is configured for connection to an external source of electrical power, e.g. a mains electrical supply outlet. The connector 106 may be used in substitution for the power source 105. That is the connector 106 may be electrically connectable to the heater 104 so as to supply electricity to the heater 104. In such embodiments, the device may not include a power source, and the power source of the system may instead comprise the connector 106 and an external source of electrical power (to which the connector 106 provides electrical connection).

In some embodiments, the connector 106 may be used to charge and recharge the power source 105 where the power source 105 includes a rechargeable battery.

The system 100 also comprises a user interface (UI) 107. Although not shown, the UI 107 may include input means to receive commands from a user. The input means of the UI 107 allows the user to control at least one aspect of the operation of the system 100. The input means may, for example, be in the form of a button, touchscreen, switch, microphone, etc.

The UI 107 also comprises output means to convey information to the user. The output means may, for example, comprise lights (e.g. LEDs), a display screen, speaker, vibration generator, etc.

The system 100 further comprises a controller 108 that is configured to control at least one function of the device 101. In the illustrated embodiment, the controller 108 is a component of the device 101, but in other embodiments may be separate from (but connectable to) the device 101. The controller 108 is configured to control the operation of the heater 104 and, for example, may be configured to control the voltage applied from the power source 105 to the heater 104. The controller 108 may be configured to toggle the supply of power to the heater 104 between an on state, in which the full output voltage of the power source 105 is applied to the heater 104, and an off state, in which no voltage is applied to the heater 104.

Although not shown, the system 100 may also comprise a voltage regulator to regulate the output voltage from the power source 105 to form a regulated voltage. The regulated voltage may then be applied to the heater 104.

In addition to being connected to the heater 104, the controller 108 is operatively connected to the UI 107. Thus, the controller 108 may receive an input signal from the input means of the UI 107. Similarly, the controller 108 may transmit output signals to the UI 107. In response, the output means of the UI 107 may convey information, based on the output signals, to a user. The controller also comprises a memory 109, which is a non-volatile memory. The memory 109 includes instructions, which, when implemented, cause the controller to perform certain tasks or steps of a method.

Figure 1B is a schematic showing a variation of the system 100 of Figure 1A. In the system 100' of Figure 1B, the heater 104 forms part of the consumable 102, rather than the device 101. In this variation, the heater 104 is electrically connectable to the power source 105, for example, when the consumable 102 is engaged with the device 101.

The systems 100, 100' of Figures 1A and 1B may be implemented as one of two broad categories of system, each in accordance with the present invention: a heated tobacco (HT) system or an e-cigarette system. A description of each category of system follows.

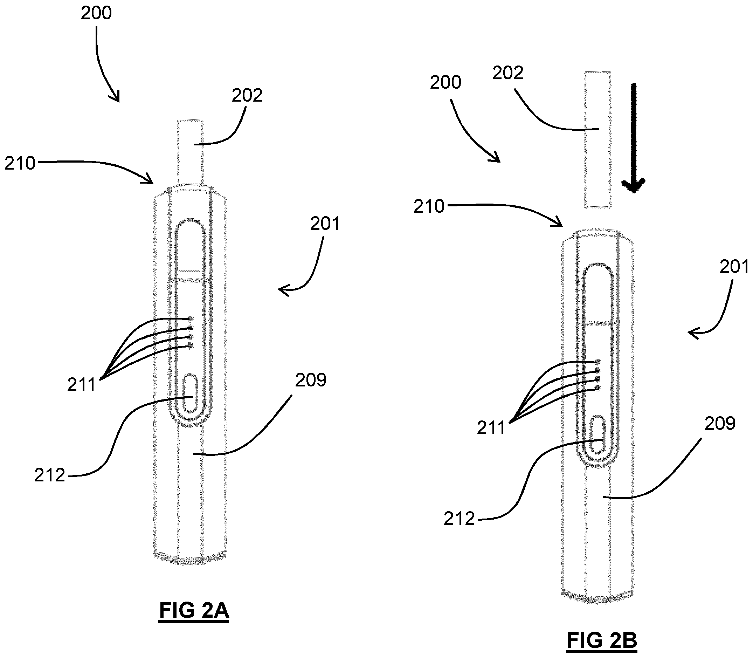

Figures 2A and 2B illustrate a heated-tobacco (HT) smoking substitute system 200. The system 200 is an example of the systems 100, 100' described in relation to Figures 1A or 1B. System 200 includes a heat- not burn (HNB) device 201 and an HT consumable 202. The description of Figures 1A and 1B above is applicable to the system 200 of Figures 2A and 2B, and will thus not be repeated.

The device 201 and the consumable 202 are configured such that the consumable 202 can be engaged with the device 201. Figure 2A shows the device 201 and the consumable 202 in an engaged state, whilst Figure 2B shows the device 201 and the consumable 202 in a disengaged state.

The device 201 comprises a body 209 and cap 210. In use the cap 210 is engaged at an end of the body 209. Although not apparent from the figures, the cap 210 is moveable relative to the body 209. In particular, the cap 210 is slideable and can slide along a longitudinal axis of the body 209.

The device 201 comprises an output means (forming part of the UI of the device 201) in the form of a plurality of light-emitting diodes (LEDs) 211 arranged linearly along the longitudinal axis of the device 201 and on an outer surface of the body 209 of the device 201. A button 212 is also arranged on an outer surface of the body 209 of the device 201 and is axially spaced (i.e. along the longitudinal axis) from the plurality of LEDs 211.

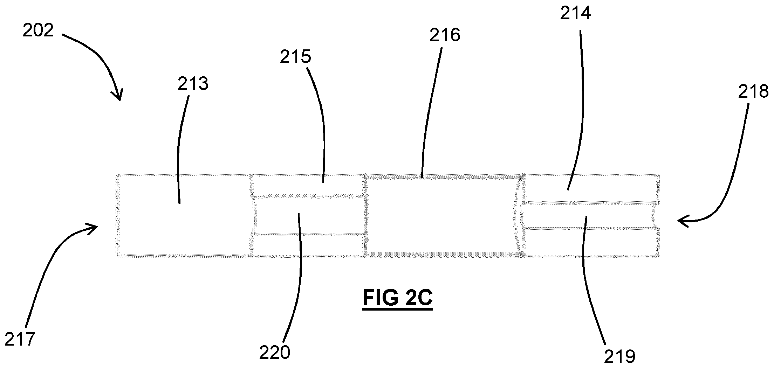

Figure 2C shows a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 214, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth end) of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 215 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14 mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

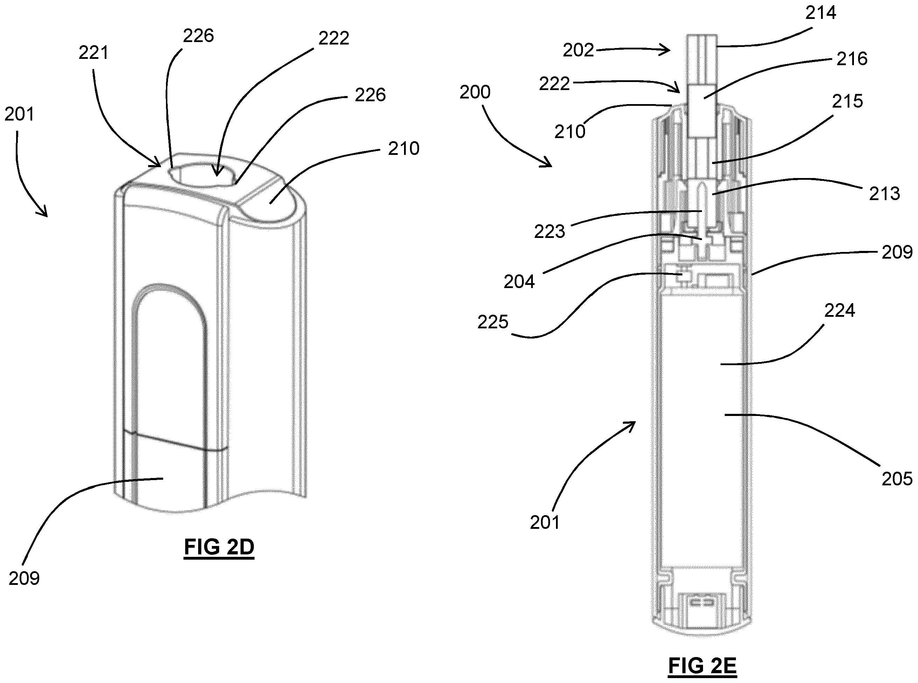

Returning now to the device 201, Figure 2D illustrates a detailed view of the end of the device 201 that is configured to engage with the consumable 202. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus also protrudes from the device 201. The opening 221 includes laterally disposed notches 226. When a consumable 202 is received in the opening 221, these notches 226 remain open and could, for example, be used for retaining a cover in order to cover the end of the device 201.

Figure 2E shows a cross section through a central longitudinal plane through the device 201. The device 201 is shown with the consumable 202 engaged therewith.

The device 201 comprises a heater 204 comprising heating element 223. The heater 204 forms part of the body 209 of the device 201 and is rigidly mounted to the body 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2E), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface the heating element 223 to the aerosol-forming substrate 213.

The device 201 further comprises an electronics cavity 224. A power source, in the form of a rechargeable battery 205 (a lithium ion battery), is located in electronics cavity 224.

The device 201 includes a connector (i.e. forming part of an IO module of the device 201) in the form of a USB port 206. The connector may alternatively be, for example, a micro-USB port or a USB-C port for examples. The USB port 206 may be used to recharge the rechargeable battery 205.

The device 201 includes a controller (not shown) located in the electronics cavity 224. The controller comprises a microcontroller mounted on a printed circuit board (PCB). The USB port 206 is also connected to the controller 208 (i.e. connected to the PCB and microcontroller).

The controller 208 is configured to control at least one function of the device 201. For example, the controller 208 is configured to control the operation of the heater 204. Such control of the operation of the heater 204 may be accomplished by the controller toggling the electrical connection of the rechargeable battery 205 to the heater 204. For example, the controller 208 is configured to control the heater 204 in response to a user depressing the button 212. Depressing the button 212 may cause the controller to allow a voltage (from the rechargeable battery 205) to be applied to the heater 204 (so as to cause the heating element 223 to be heated).

The controller is also configured to control the LEDs 211 in response to (e.g. a detected) a condition of the device 201 or the consumable 202. For example, the controller may control the LEDs to indicate whether the device 201 is in an on state or an off state (e.g. one or more of the LEDs may be illuminated by the controller when the device is in an on state).

The device 201 comprises a further input means (i.e. in addition to the button 212) in the form of a puff sensor 225. The puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing), forms an input to the controller 208 (and can thus be responded to by the controller 208).

Figures 3A to 3E illustrate a smoking substitute device which is fully in accordance with the present invention as defined in claims 1 and 12, said device having a closure wherein the closure 250 may be a cylindrical shaped member having a bore 254 therethrough. The closure 250 is mounted concealed within the body 209 such that the closure 250 rotates between a first position and a second position, as illustrated in Figs. 3A/C and Figs. 3B/D. The bore is a through-hole perpendicular to an axis of rotation of the closure 250. When the closure 250 is in the second position, the bore is aligned along with longitudinal axis of the body 209 such that the bore 254 and cavity 222 provide a passage for the insertion of a consumable 202 through the opening 221. When the closure 250 in first position the bore 254 is aligned in a direction which is not parallel with the cavity 222, thereby closing the opening 221 of the cavity 222.

Further, the closure 250 or ball valve may include a handle 260, shown in Fig. 3E, allowing the user to move the closure 250 between the first position and the second position. E.g. the user may hold the smoking substitute device 201 and operate the handle with one's thumb, thereby providing a one hand operation. The position may trigger a switching on/off of the smoking substitute device and/or the operation of the device, to allow a pure one hand operation. In an embodiment, a portion of the ball valve may be exposed to an outer surface of the body 250 to facilitate the user to rotate the ball valve manually between the first position and the second position. Furthermore, the closure 250 may be biased into one or more of the first and second positions.

In some arrangements which may fall outside the scope of the invention, , the closure comprises a swinging closure, such as a concealed trap door within the device body. For example, in arrangements that fall outside the scope of claim 1 the closure may comprise a hinged sheet of material which is biased into the first position (closed) in which the sheet covers the opening, wherein when force is applied to the sheet in a direction into the device to overcome the bias, the sheet swings via the hinge into the second position (open), allowing insertion of a consumable into the cavity. In this way, the user is able to open the closure simply by pressing the end of a consumable against the sheet, into the device, which pushes the closure away from the opening to allow the consumable to pass into the device. In some such arrangements, the trap door is biased into the first position (closed). For example, the trap door may be spring-loaded.

In some embodiments, the device comprises means to hold the closure in one or more of the first position and the second position. In some embodiments, the means to hold the closure comprises an interaction between the closure and a part of the body of the device which occurs at or close to the first and/or second position. In some embodiments, the means to hold the closure comprises a detent comprising a raised feature on a surface of the device body and/or the closure. In some embodiments, the means to hold the closure comprises an interference fit provided between the closure and the body of the device when in the first and/or second positions, wherein the interference fit is removed as the closure moves away from the first and/or second position to facilitate movement between the positions.

The heat-not-burn device 201 also comprises a sensor (not shown in figures) for detecting a position of the closure 250. The sensor is communicatively coupled with the controller 208 to receive a signal from the sensor.

Further, the controller 208 is configured to receive the signal from the sensor, indicative of a position of the closure 250. Based on the position of the closure 250, the controller 208 controls activation and deactivation of heater 204 in response to the received signal. The sensor may detect the first position and the second position of the closure 250. The sensor may generate a signal based on the determination of position of the closure 250 in the first position. Further, upon receiving the signal from the sensor, the controller 208 may deactivate the power supply to the device 201, thereby preventing activation of the heater 204. Similarly, the sensor is configured to generate another signal, based on the determination of the position of the closure 250 in the second position. Consequently, the controller 208 may activate the heater 204 for heating the consumable 202 received within the cavity 222. In this way, the heater cannot be activated when the closure 250 is "closed". This provides a safer and more efficient device since accidental activation of the heater 204 e.g. in a pocket or bag is prevented, which saves battery life and is safer. When the user opens the closure 250, the controller 208 then permits the activation of the heater 204 (e.g. by an appropriate input on a user interface).

In another aspect the present invention discloses a method of operating a heat not burn device 201, the method comprises steps of determining a position of a closure 250 for covering an opening 221 of the device 201 into which a heat-not-burn consumable 202 is received in use. Secondly, a heater 204 of the device 201 is controlled based on the determined position of the closure 250. The position of the closure 250 is determined by at least one sensor (not shown in figures) disposed in the device 201.

Further, the method of operating the heat not burn 201 may comprise disabling activation of the heater 204. The activation and disabling activation of the heater 204 may be controlled by a controller 208, based on the position of the closure 250. The position of the closure 250 may be detected by a sensor configured within the device 201 and communicatively coupled with the controller 208.

In another aspect, the power supply (i.e., power source) of the device may be disabled by the controller 208 when the closure 205 is in the first position. The disabling activation of the device 201 based on the position of the closure 250 may facilitate optimum working of the device 201. The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised.

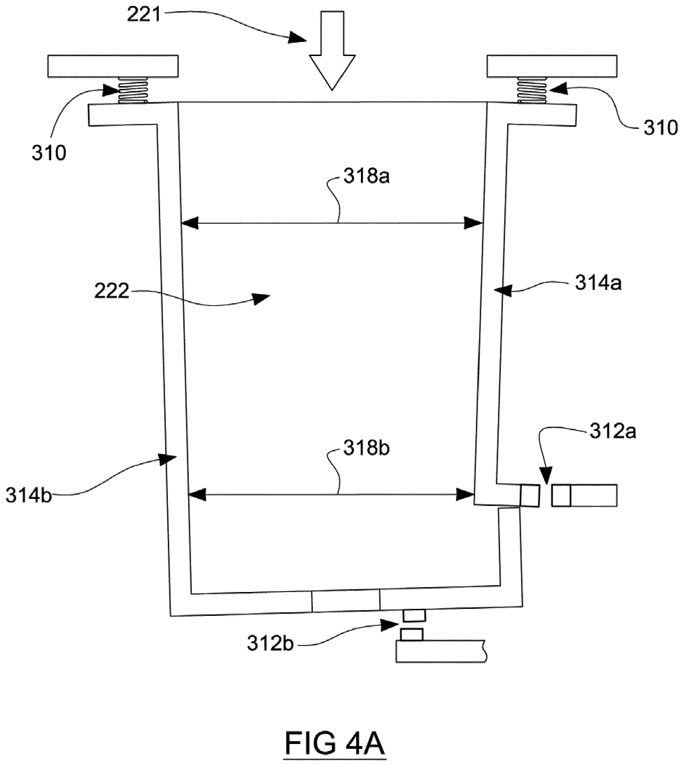

Turning now to consider Figure 4A, there is illustrated a detailed view of a smoking substitute device 201 with no consumable inserted in the smoking substitute device 201.

The cavity 222 exemplarily comprises two deformable walls 314a,b. The cavity 222 is accessible via opening 221 for insertion of a consumable 102, as symbolized by the downward arrow, but not depicted in Figure 4A. Exemplarily, the cavity 222 is generally V-shaped in Figure 4A, since no consumable is inserted and the deformable walls 314a,b assume a first position relative one another and relative to the other elements in the smoking substitute device 201, like the top part comprising the opening 221. This V-shape is symbolized by the size of two cross-section of the cavity 318a,b, one cross-section 318a near the opening 221 and one cross-section 318b near the bottom of the cavity 222. Here, in the absence of a consumable 202 in the cavity 222, cross section 318a is larger than the cross-section 318b.

Figure 4A shows two detection element 312a,b. Detection element 312a is arranged adjacent to deformable wall 314a while detection element 312b is arranged adjacent to deformable wall 312b. As may be taken from Figure 4A, the detection elements 312a,b are not in contact with the deformable walls 314a,b. The deformable walls 314a,b are connected to the smoking substitute device 201 by spring element 310, basically suspending the cavity 222 in the interior of the smoking substitute device 201.

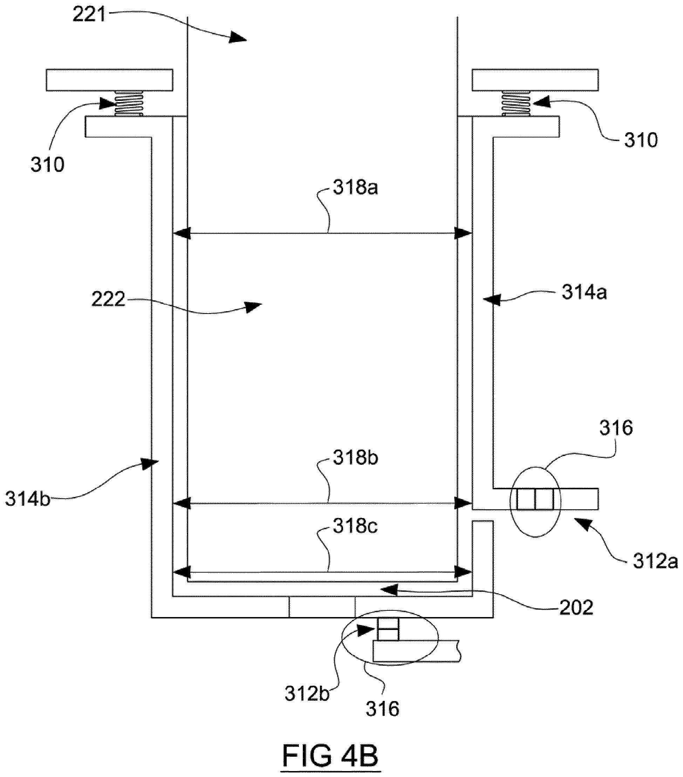

Figure 4B is a detailed view of a smoking substitute device 201 with a consumable 222 inserted in the smoking substitute device 201. In Figure 4B, the deformable walls 314a,b are arranged substantially parallel along the longitudinal extension of the smoking substitute device 201, since a consumable 202 is present in the cavity 222. When the consumable 202 is inserted into the cavity 222, the deformable walls are deformed, i.e. pushed aside, so to assume a different position relative one another and relative to other elements on the smoking substitute device 201. As a consequence, different cross-sections 318a,b,c are now substantially similar and in particular conform to the diameter of the consumable 202. By the insertion of the consumable, the deformable walls 314a,b are moved towards the detection elements 312a,b and are now contacting 316 the detection elements 312a,b.

The detection elements 312a,b may now be arranged to either detect the contacting 316 on their own and in particular independently from one another, and may relay said information to a controller of the smoking substitute device 201, in order to enable the controller to detect or ascertain said contacting 316 and thus the (correct) insertion of the consumable 202. In other words, the insertion of the consumable 202 may provide a shifting or rotation of deformable wall 314a in the direction of detection element 312a, and thus the contacting 316 of the detection element 312a.

Further, the insertion of the consumable 202 in a depth direction of the cavity 222 may affect the contacting of the deformable wall 314b and the detection element 312b. E.g. in case the consumable 202 is not inserted sufficiently deep into the cavity, the detection element 312b may not detect the proper insertion, since the contact 316 has not yet been established. The spring elements 310 may allow the deformable walls 314a,b to sufficiently change their position within the smoking substitute device 201to allow connecting 316 the detection element 312a,b.

With the connection 316 of the deformable walls 314a,b to the detection elements 312a,b, an electrical circuit may be closed, either within each detection element 312a,b when contacted by the deformable wall 314a,b, or a connection between the two detection elements 312a,b via, e.g. a conductive path, e.g. along the surface of the deformable walls 314a,b, which closes an electric circuit the between detection elements 312a.

Likewise, the detection elements may detect the deformation or change in position of the deformable walls in a non-contacting way, e.g. by magnetic detection elements detecting the closeness of the deformable walls. Such detection elements 312a,b may e.g. be implemented as reed switches, and the magnetic counterpart may be at least forming part of a deformable wall. Likewise, the deformable wall may be made of a material that allows triggering the reed switch when sufficiently close, e.g. when the consumable 202 in inserted in the cavity 222.

The exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.
The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful.

## Claims

1. A smoking substitute device (101,201) comprising
a body (209) having a cavity (222) for receiving of an aerosol-generating consumable (202);
a closure (250) moveable by rotatory movement between a first position in which it covers the cavity (222) and a second position in which the cavity (222) is substantially uncovered, and
a handle (260) for moving the closure (250) between the first and the second position
**characterised in that**:
the closure (250) comprises a through-hole bore (254) perpendicular to an axis of rotation of the closure (250),
wherein the through-hole bore (254) aligns with the cavity (222) to provide a passage for inserting an aerosol-generating consumable (202) in the cavity (222) when the closure (250) is in the second position.

2. The device (101,201) according to any of the preceding claims, wherein the device (101,201) comprises means to hold the closure (250) in one or more of the first position and the second position.

3. The device (101,201) according to the preceding claim, wherein the means to hold the closure (250) is at least one means out of the group consisting of a detent comprising a raised feature on a surface of the device body (209), a magnet or a spring.

4. The device (101,201) according to any of the preceding claims, wherein the closure (250) is provided with the handle (260) external to the body (209) of the device (101,201) for allowing a user to move the closure (250) between the first position and the second position.

5. The device (101,201) according to any of the preceding claims, wherein the closure (250) is a made of flexible material.

6. The device (101,201) according to any of the preceding claims, further comprising
a sensor for detecting a position of the closure (250);
a heater (104,204) for heating the consumable (202) when received in the cavity (222); and a controller (208) configured to receive a signal from the sensor, indicative of a position of the closure (250), and to control the heater (104,204) in response to the received signal.

7. The device (101,201) according to the preceding claim, wherein the sensor is configured to generate a signal upon detecting that the closure (250) is in the first position, and wherein the controller (208) deactivates the heater (104,204) based on the received signal.

8. The device (101,201) according to any of the preceding claims, wherein the closure (250) comprises biasing means which urge the closure (250) into one or both of the first position and the second position.

9. The device according to the preceding claim, wherein the biasing means comprises a magnet or spring.

10. The device according to any of the preceding claims, wherein the closure (250) is interposed between an opening to the cavity (222) and a rod heater (104,204), wherein the rod heater (104,204) is disposed within the cavity (222) along a longitudinal axis of the body (209).

11. A smoking substitute system, comprising a smoking substitute device (101,201) according to any of the preceding claims and an aerosol generating consumable (202).

12. A method of operating a smoking substitute device (101,201), according to any of the preceding claims 1 to 10, the method comprising:
determining a position of a closure (250) for covering a cavity (222) of the device into which an aerosol generating consumable (202) is received in use; and
controlling a heater (104,204) of the device (101,201) based on the determined position of the closure (250).

13. The method of the preceding claim, wherein controlling the heater (104,204) of the device (101,201) comprises
determining, by a sensor associated with the controller (208), the position of the closure (250), wherein the controller (208) deactivates the heater (104,204) based on a signal received from the sensor when the closure (250) is in a first position where the closure (250) covers a cavity (222) in the device (101,201).

## Patentansprüche

1. Rauchersatzvorrichtung (101, 201), umfassend:
einen Körper (209) mit einem Hohlraum (222) zum Aufnehmen einer ein Aerosol erzeugenden Verbrauchsware (202);
einen Verschluss (250), der durch eine Drehbewegung zwischen einer ersten Position, in der er den Hohlraum (222) bedeckt, und einer zweiten Position, in welcher der Hohlraum (222) im Wesentlichen nicht bedeckt ist, bewegbar ist, und
einen Griff (260) zum Bewegen des Verschlusses (250) zwischen der ersten und der zweiten Position,
**dadurch gekennzeichnet, dass**
der Verschluss (250) eine Durchgangslochbohrung (254) senkrecht zu einer Drehachse des Verschlusses (250) umfasst,
wobei die Durchgangslochbohrung (254) mit dem Hohlraum (222) fluchtend angeordnet ist, um einen Durchgang zum Einsetzen einer ein Aerosol erzeugenden Verbrauchsware (202) in den Hohlraum (222) bereitzustellen, wenn sich der Verschluss (250) in der zweiten Position befindet.

2. Vorrichtung (101, 201) nach einem der vorangegangenen Ansprüche, wobei die Vorrichtung (101, 201) ein Mittel zum Halten des Verschlusses (250) in einer oder mehreren aus der ersten Position und der zweiten Position umfasst.

3. Vorrichtung (101, 201) nach dem vorangegangenen Anspruch, wobei das Mittel zum Halten des Verschlusses (250) zumindest ein Mittel aus der Gruppe bestehend aus einer Arretierung, die ein erhöhtes Merkmal auf einer Oberfläche des Vorrichtungskörpers (209) umfasst, einem Magneten oder einer Feder ist.

4. Vorrichtung (101, 201) nach einem der vorangegangenen Ansprüche, wobei der Verschluss (250) mit dem Griff (260) außerhalb des Körpers (209) der Vorrichtung (101, 201) bereitgestellt ist, um es einem Benutzer zu ermöglichen, den Verschluss (250) zwischen der ersten Position und der zweiten Position zu bewegen.

5. Vorrichtung (101, 201) nach einem der vorangegangenen Ansprüche, wobei der Verschluss (250) aus einem flexiblen Material besteht.

6. Vorrichtung (101, 201) nach einem der vorangegangenen Ansprüche, ferner umfassend
einen Sensor zum Detektieren einer Position des Verschlusses (250);
ein Heizelement (104, 204) zum Erhitzen der Verbrauchsware (202), wenn sie im Hohlraum (222) aufgenommen ist; und
eine Steuerung (208), die dazu ausgelegt ist, ein Signal vom Sensor zu empfangen, welches eine Position des Verschlusses (250) angibt, und das Heizelement (104, 204) als Antwort auf das empfangene Signal zu steuern.

7. Vorrichtung (101, 102) nach dem vorangegangenen Anspruch, wobei der Sensor dazu ausgelegt ist, ein Signal zu erzeugen, wenn er detektiert, dass der Verschluss (250) sich in der ersten Position befindet, und wobei die Steuerung (208) das Heizelement (104, 204) beruhend auf dem empfangenen Signal deaktiviert.

8. Vorrichtung (101, 201) nach einem der vorangegangenen Ansprüche, wobei der Verschluss (250) ein Vorspannmittel umfasst, das den Verschluss (250) in eine oder beide aus der ersten Position und der zweiten Position drückt.

9. Vorrichtung nach dem vorangegangenen Anspruch, wobei das Vorspannelement einen Magneten oder eine Feder umfasst.

10. Vorrichtung nach einem der vorangegangenen Ansprüche, wobei der Verschluss (250) zwischen einer Öffnung zum Hohlraum (222) und einem Stabheizelement (104, 204) angeordnet ist, wobei das Stabheizelement (104, 204) innerhalb des Hohlraums (222) entlang einer Längsachse des Körpers (209) angeordnet ist.

11. Rauchersatzsystem, umfassend eine Rauchersatzvorrichtung (101, 201) nach einem der vorangegangenen Ansprüche und eine ein Aerosol erzeugende Verbrauchsware (202).

12. Verfahren zum Betreiben einer Rauchersatzvorrichtung (101, 201) nach einem der vorangegangenen Ansprüche 1 bis 10, wobei das Verfahren Folgendes umfasst:
Bestimmen einer Position eines Verschlusses (250) zum Bedecken eines Hohlraums (222) der Vorrichtung, in dem im Gebrauch eine ein Aerosol erzeugende Verbrauchsware (202) angeordnet ist; und
Steuern eines Heizelements (104, 204) der Vorrichtung (101, 201) beruhend auf der bestimmten Position des Verschlusses (250).

13. Verfahren nach dem vorangegangenen Anspruch, wobei das Steuern des Heizelements (104, 204) der Vorrichtung (101, 201) Folgendes umfasst:
Bestimmen der Position des Verschlusses (250) durch einen der Steuerung (208) zugeordneten Sensor, wobei die Steuerung (208) das Heizelement (104, 204) beruhend auf einem vom Sensor empfangenen Signal deaktiviert, wenn sich der Verschluss (250) in einer ersten Position befindet, in der der Verschluss (250) einen Hohlraum (222) in der Vorrichtung (101, 201) bedeckt.

## Revendications

1. Dispositif à fumer de substitution (101, 201), comprenant :
un corps (209) présentant une cavité (222) pour recevoir un consommable de génération d'aérosol (202) ;
une fermeture (250) mobile par l'intermédiaire d'un déplacement rotatif entre une première position dans laquelle elle recouvre la cavité (222) et une seconde position dans laquelle la cavité (222) est sensiblement découverte, et
une poignée (260) pour déplacer la fermeture (250) entre la première et la seconde position, **caractérisé en ce que** :
la fermeture (250) comprend un alésage traversant (254) perpendiculaire à un axe de rotation de la fermeture (250),
dans lequel l'alésage traversant (254) s'aligne avec la cavité (222) pour fournir un passage pour insérer un consommable de génération d'aérosol (202) dans la cavité (222) lorsque la fermeture (250) est dans la seconde position.

2. Dispositif (101, 201) selon l'une quelconque des revendications précédentes, dans lequel le dispositif (101, 201) comprend un moyen pour maintenir la fermeture (250) dans une ou plusieurs parmi la première position et la seconde position.

3. Dispositif (101, 201) selon la revendication précédente, dans lequel le moyen pour maintenir la fermeture (250) est au moins un moyen du groupe constitué d'une détente comprenant une caractéristique surélevée sur une surface du corps de dispositif (209), un aimant ou un ressort.

4. Dispositif (101, 201) selon l'une quelconque des revendications précédentes, dans lequel la fermeture (250) est pourvue de la poignée (260) externe au corps (209) du dispositif (101, 201) pour permettre à un utilisateur de déplacer la fermeture (250) entre la première position et la seconde position.

5. Dispositif (101, 201) selon l'une quelconque des revendications précédentes, dans lequel la fermeture (250) est réalisée en un matériau flexible.

6. Dispositif (101, 201) selon l'une quelconque des revendications précédentes, comprenant en outre
un capteur pour détecter une position de la fermeture (250) ;
un dispositif de chauffage (104, 204) pour chauffer le consommable (202) lorsqu'il est reçu dans la cavité (222) ; et
un dispositif de commande (208) configuré pour recevoir un signal provenant du capteur, indiquant une position de la fermeture (250), et pour commander le dispositif de chauffage (104, 204) en réponse au signal reçu.

7. Dispositif (101, 201) selon la revendication précédente, dans lequel le capteur est configuré pour générer un signal lors d'une détection que la fermeture (250) est dans la première position, et dans lequel le dispositif de commande (208) désactive le dispositif de chauffage (104, 204) sur la base du signal reçu.

8. Dispositif (101, 201) selon l'une quelconque des revendications précédentes, dans lequel la fermeture (250) comprend des moyens de sollicitation qui poussent la fermeture (250) dans l'une ou les deux de la première position et de la seconde position.

9. Dispositif selon la revendication précédente, dans lequel le moyen de sollicitation comprend un aimant ou un ressort.

10. Dispositif selon l'une quelconque des revendications précédentes, dans lequel la fermeture (250) est interposée entre une ouverture de la cavité (222) et un dispositif de chauffage à tige (104, 204), dans lequel le dispositif de chauffage à tige (104, 204) est disposé à l'intérieur de la cavité (222) le long d'un axe longitudinal du corps (209).

11. Système à fumer de substitution, comprenant un dispositif à fumer de substitution (101, 201) selon l'une quelconque des revendications précédentes et un consommable de génération d'aérosol (202).

12. Procédé de fonctionnement d'un dispositif à fumer de substitution (101, 201) selon l'une quelconque des revendications précédentes 1 à 10, le procédé comprenant les étapes consistant à :
déterminer une position d'une fermeture (250) pour couvrir une cavité (222) du dispositif dans laquelle un consommable de génération d'aérosol (202) est reçu en cours d'utilisation ; et
commander un dispositif de chauffage (104, 204) du dispositif (101, 201) sur la base de la position déterminée de la fermeture (250).

13. Procédé selon la revendication précédente, dans lequel la commande du dispositif de chauffage (104, 204) du dispositif (101, 201) comprend l'étape consistant à
déterminer, par l'intermédiaire d'un capteur associé au dispositif de commande (208), la position de la fermeture (250), dans lequel le dispositif de commande (208) désactive le dispositif de chauffage (104, 204) sur la base d'un signal reçu à partir du capteur lorsque la fermeture (250) est dans une première position où la fermeture (250) recouvre une cavité (222) dans le dispositif (101, 201).
